# EUROPEAN PATENT APPLICATION

(11) **EP 4 599 691 A1**
(43) Date of publication of application: **13.08.2025**
(21) Application number: 23875299.2
(22) Date of filing: 29.09.2023
(51) Int. Cl.: A23L 2/54, A23L 33/10, A23L 3/37

(54) **METHOD FOR IMPROVING THE BIOAVAILABILITY OF FUNCTIONAL FOOD PRODUCTS**

(30) Priority: 07.10.2022 RU 2022126213
(71) Applicant: Noble Boost B.V., 6229 ES Maastricht (NL)
(72) Inventor: VERKHOVSKY, Aleksandr Yurevich, Tomsk, 634061 (RU)
(74) Representative: Koudine, Andreï
(86) International application number: PCT/RU2023/050230
(87) International publication number: WO 2024/076263

(57) **Abstract**

The invention relates to the food and pharmaceutical industry, namely to food products, functional nutrition products, dietary supplements, beverages, and represents a method for improving the digestibility of functional nutrition products using an inert gas. The method for improving the digestibility of products using the inert gas includes a mixing of the inert gas with a functional nutrition product, wherein the functional nutrition products used are a fat emulsion, oils, water-soluble ingredients used in manufacturing of nutrition products, dietary supplements, beverages, and the active ingredient is at least one gas from the group including xenon, krypton, the gas content in the nutrition product being from 5 ml/l to 2,8 l/l, or a mixture thereof in various ratios. The use of the invention makes it possible to enhance the digestion of functional nutrition products.

## Description

The invention relates to the food and pharmaceutical industry, namely to food products, functional nutrition products, dietary supplements, beverages and represents a method for improving the bioavailability of functional nutrition products using an inert gas.

Recently, modern achievements of science and technology have been increasingly used in food manufacturing. These include, for example, the results of research on the use of inert gases.

Inert gases or their mixtures protect the food product from environmental influences. Protective inert gases approved for use in food and beverage manufacturing include: nitrogen, argon, helium, xenon, krypton (B.L. Krasny, V.P. Tarasovsky, A.B. Krasny, Y.G. Matytsin, N.A. Tikhomirova, Y.A. Likhachev, A.V. Potapov. Inert gases in food industry: prospects of use. URL: https://inlnk.ru/MjK4Qn).

An agent for increasing the performance capacity of an organism based on α-cyclodextrin and its application are known (patent RU2704024, A61K 31/724, A61K 33/00, A61P 43/00, published on 23.10.2019). The mammalian body performance increasing agent comprising a first component which is a lyophilized α-cyclodextrin-based powder, which is obtained as follows: in a first step saturating α-cyclodextrin with xenon in an aqueous solution at a temperature from 0 to 35°C, in a second step dehydrating the xenon-saturated α-cyclodextrin at a temperature from 0 to -70°C; a second component representing a liquid edible oil; a ratio of the first component to the second component being from 1 to 6 to 1 to 7, respectively. A method for increasing the performance capacity of a mammalian body. This patent describes a method of delivering an inert gas using a two-component carrier, we have a different technical result, namely, the gas is used as a means for enhancing the digestibility of other bioactive products.

A preparation for adaptogenic therapy and a method of its manufacturing are known (patent 2228739, published 20.05.2004, A61K9/107). The invention relates to the field of pharmaceutics and concerns a preparation for adaptogenic therapy and a method of production thereof. The invention consists in that the preparation is provided with a hermetically sealed package and comprises a filler - a fat emulsion or a solid sorbent, in which a gaseous active ingredient is dissolved or adsorbed, which is at least one gas from the group including: xenon, krypton, nitrous oxide. Preferably, the filler is a fat emulsion having a pH of at least 6,0, the fat emulsion comprises a fat, namely bovine or small cattle milk or other emulsion based on animal oil, or as a filler it comprises vegetable oil or activated carbon or thermoxide. The invention is directed to obtain a non-toxic preparation with increased ability to regulate the body's resistance under extreme conditions through humoral regulation, metabolic regulation and psycho-emotional regulation of the patient. Said patent is directed to a method of delivering gases to the body using sorbents in a hermetically sealed shell.

The objective of the present invention is to provide a method for improving the bioavailability of functional nutrition products using an inert gas.

The set task is solved by a method for improving the bioavailability of products using an inert gas, said method including a mixing the inert gas with a functional nutrition product, but - in contrast to the prototype - the functional nutrition products used are a fat emulsion, oils, oil-soluble, water-soluble insoluble ingredients used in manufacturing of nutrition products, dietary supplements, beverages, and the active ingredient represents at least one gas from the group including xenon, krypton, the gas content in the nutrition product being from 5 ml/l to 2,8 l/l, or a mixture thereof in various ratios.

Once a gas supply stopped, the nutrition product is packaged in a hermetically sealed packaging (metal, plastic, glass, gelatin capsules and so on).

For an inert gas saturation of the functional product no overpressure is used, or an overpressure of up to 2 atm is used.

The functional nutrition product is made in a liquid, soft, semi-soft form.

Krypton is an inert, colorless, tasteless and odorless one-atom gas. It is obtained from krypton-xenon mixture in the process of air separation at industrial plants. During an air separation by a low-temperature rectification method, a liquid oxygen fraction comprising liquid hydrocarbons, krypton and xenon is continuously extracted (extraction of the oxygen fraction with hydrocarbons is necessary to ensure an explosion safety). To extract krypton and xenon from the selected fraction, hydrocarbons are removed in catalytic furnaces and sent to an additional rectification column for oxygen removal; once Kr+Xe mixture is enriched up to 98-99%, it is repeatedly purified in catalytic furnaces from hydrocarbons and, after that, in adsorber block filled with silica gel (or other adsorbent). After purification of the gas mixture from hydrocarbon residues and moisture, it is pumped into cylinders for transportation to the plants for separation and purification of Kr and Xe.

Xenon is an inert noble gas, absolutely non-toxic; it does not react with biological molecules, does not exhibit mutagenic, teratogenic, cyto- and immunotoxic properties. It is used in medicine since 1951 for inhalation anaesthesia. When injected into the body, the gas is completely eliminated within 4 hours (Sanders R.D., Franks N.P., Maze M. Xenon: no stranger to anaesthesia / Brit J Anaesthesia, 2003. - V.91 (5). - P.709-717).

A bioavailability (BA) is a degree of absorption of a drug substance from the site of administration into a systemic bloodstream and a rate at which this process occurs (Khoruzhaya T.G., Chuchalin V.S. Biopharmacy - scientific direction in the development and improvement of drugs. 2006.). However, the concept of bioavailability includes not only the rate and degree of absorption of the active substance, but also its other features: availability of the active substance and formed metabolites at the site of action. This is supported by the following definitions: the bioavailability is the degree and rate at which the active drug substance is absorbed from a pharmaceutical form and becomes available at the site of action (Toutain P.L., Bousquet-Me, Lou A.. Bioavailability and its assessment. // J.vet. Pharmac. Therapy. 2004. V.27. P. 455-466. Oral bioavailability: prediction using in vitro kinetic data. Kinetics and Metabolism. 2009. Guidance for Industry, Bioavailability and Bioequivalence Studies for Orally Administered Drugs, Products - General Considerations. U.S.Department of Health and Human Services Food and Drug Administration, Centre for Drug Evaluation and Research (CDER). 2003.). The bioavailability is a part of an ingested dose of a medicinal substance, which reached the systemic bloodstream in unchanged form and in the form of active metabolites formed in the process of absorption and presystemic metabolism (Khoruzhaya T.G., Chuchalin V.S. Biopharmacy - scientific direction in the development and improvement of drugs. 2006.).

The bioavailability of drugs is influenced by so-called endogenous factors. These include: physiological factors: age, sex, state of the patient's body; biochemical factors: state of cell membranes, cell activity, presence of endogenous substrates accumulated in various diseases (bilirubin, fatty acids, etc.); pathophysiological factors: pathological conditions of the gastrointestinal tract, liver, kidneys, heart, etc.); pathophysiological: pathological conditions of the gastrointestinal tract, liver, kidneys, cardiovascular system, level of transport proteins in the blood, genetically determined difference in biotransformation of drugs with presystemic metabolism; clinical factors: choice of dosing scheme, route of administration, site of injection, interaction of simultaneously or sequentially administered drugs (Tikhonov A.I., Yarnykh T.G., Zupanets I.A. Biopharmacy. Publishing house "Golden Pages". 2003.).

Examples of specific uses of the invention are given below.

### Example 1. Effect of xenon and krypton on the digestibility of olive oil triglycerides.

Studies on the effect of xenon on the absorption and transport of triacylglycerols were carried out in an experiment as described [Wang Z., Wang L., Zhang Z., Feng L., Song X., & Wu J. Apolipoprotein A-IV involves in glucose and lipid metabolism of rat //Nutrition & metabolism. - 2019. - T. 16. - 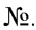 1. - P. 1-9.], were performed on 12 Wistar rats weighing 320-360 g.

All animals were divided into 3 groups: 1 - control, 2 - experimental, 3 - experimental. The animals were fasted for 6 hours before the experiment, and then rats of the control group (n=4) were injected intragastrically with bolus Extra Virgin olive oil (Aceites Toledo, Spain) at a dose of 1 ml/100 g body weight. This amount of oil is sufficient for the induction of postprandial increase in the level of triacylglycerols in plasma in rats and to assess the absorption of fats without the use of radioactive tags [Martínez-Beamonte R., Navarro M. A., Acin S., Guillen N., Barranquero C., Arnal C., Osada J. Postprandial changes in high density lipoproteins in rats subjected to gavage administration of virgin olive oil //PLoS One. - 2013. - T. 8. - 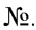 1. - P. e55231.]. Animals of experimental group 1 (n=4) were administered olive oil saturated with xenon at a concentration of 280 vol.%, and experimental group 2 (n=4) were administered olive oil saturated with krypton at a concentration of 24 vol.%. Xenon and krypton were used here and further on with a special purity produced by "Akela-N" Ltd.

An introduction of gases into the oils was carried out using an emulsion production reactor with a volume of 11. The process was carried out at a normal pressure in a sealed condition. Gas (xenon, krypton) from a syringe through a tube was introduced from below into an area of the homogeniser immersed in oil. Gas injection was continued until the pressure increased to 0,1 mPa with the homogeniser running. A content of xenon and krypton in the oil was determined by a gas chromatography method. Before and 30 min, 1, 2 and 3 h after the olive oil injection, blood samples of 50 µl each were taken from a tail vein of rats in a Microvette CB 300 (Microvette CB 300, USA). Blood samples were centrifuged at 3000 rpm at 40°C during 10 min on a HERMLE Z383K centrifuge (Germany).

Triacylglycerols (TAG) content was immediately determined in blood serum using enzyme kits from Chronolab (Spain). For this purpose, 10 µl of serum or standard TAG solution was added to 1 ml of a working reagent, incubated for 5 min at 370°C in a thermostat and an optical density at 500 nm was determined on a spectrophotometer. Table 1 shows the effect of xenon and krypton on triacylglycerols (TAG) content in rat serum after the bolus intragastric administration of olive oil (1 ml per 100 g body weight), X ± SD.

**Table 1**

| Time | TAG content, mmol/l | | |
|---|---|---|---|
| | 1. Control (n=4) | 2. O-1(He) (n=4) | 3. O-2(Kr) (n=4) |
| 0 min. | 1,17 ± 0,31 | 1,45 ± 0,60 | 1,26 ± 0,31 |
| 30 min | 1,32 ± 0,39 | 2,35 ± 0,92 | 1,76 ± 0,90 |
| 60 min | 1,58 ± 0,39 | 2,94 ± 0,46 | 2,68 ± 1,05 |
| 120 min. | 2,77 ± 0,60 | 4,29 ± 0,82 | 4,60 ± 0,79 |
| 180 min | 2,70 ± 0,46 | 5,02 ± 0,88 | 4,81 ± 1,04 |

The results showed that xenon and krypton oil resulted in a significant increase in TAG levels 1, 2, and 3 hours after the bolus intragastric administration of olive oil. Moreover, this effect was almost the same for xenon at a concentration of 280 vol.%, and for krypton at a concentration of 24 vol.%.

### Example 2. Effect of xenon and krypton on digestibility of Omega-3 triglycerides and polyprenols from Siberian fir needles.

Studies on the effect of xenon + krypton gas mixture on absorption and transport of triacylglycerols were carried out as described above in two groups of animals. In the control group (n - 4) and experimental group (n - 4) Omega-3, BASF PronovaPure^{®} 500:200 TG (EPA mg/g - min. 500, DHA mg/g - min. 200, EPA&DHA mg/g - min. 700) with polyprenols were used. The mixture was prepared as follows: a coniferous extract (production of LLC "Siberian Natural Product") was added to Omega-3 at a concentration of 5.0% with a 48% content of polyprenols. Xenon and krypton were introduced as described above. The xenon content was 150 vol.% and krypton 13 vol.%. Daily urine was collected in the control and experimental groups to determine polyprenols (dolichols) by HPLC. Table 2 shows the effect of control and experimental samples on triacylglycerols (TAG) content in rat serum after their bolus intragastric administration (1 ml per 100 g body weight), X ± SD. Table 3 shows the effect of control and experimental samples on the content of polyprenols in the urine of rats.

**Table 2**

| Time | TAG content, mmol/l | |
|---|---|---|
| | 1. Control (n=4) | 2. O (He+Kr) (n=4) |
| 0 min. | 0,80 ±0,15 | 0,91 ± 0,28 |
| 30 min | 1,31 ± 0,25 | 1,69 ± 0,50 |
| 60 min | 2,13 ± 0,46 | 3,19 ± 0,45 |
| 120 min. | 2,49 ± 0,35 | 4,37 ± 0,62 |
| 180 min | 2,55 ± 0,56 | 4,45 ± 0,55 |

**Table 3**

| Group | Mass concentration of polyprenols (dolichols), µg/ml |
|---|---|
| Control | 6,8 ± 0,18 |
| Experience | 35,5 ± 1,2 |

The experimental results showed an approximately 2-fold increase in the absorption of Omega-3 triglycerides at 1, 2 and 3 hours after the bolus intragastric administration.

At the same time the concentration of polyprenols (dolichols) in daily urine increased about 6 times, which indicates the high efficiency of the effect of xenon and krypton mixture on the assimilation and metabolism of polyprenols.

### Example 3. Effect of xenon and krypton on the increase of Omega-3 index

Testing was carried out on 14 male volunteers, age from 38 to 47 years. Control and 5 experimental groups of 2 people each. All volunteers had an Omega-3 index of less than 4,5%. For testing the control group, the product used was Epax Ultra Concentrates (Epax 5025 TGN), manufactured by Epax Norway AS. For experimental group 1 xenon was introduced into the product in concentration of 5 vol.%; for experimental group 2 - 20 vol.%; for experimental group 3 - 250 vol.%; for experimental group 4 - krypton - 5 vol.%; for experimental group 5 - 24 vol.%; for experimental group 6 - 70 vol.%. A product saturation with gases was done as described above, but at a small overpressure: krypton at 2,0 mPa, and xenon - 0,2 mPa. All products (for control and experimental groups) were encapsulated in soft gelatin capsules weighing 0,5g. All volunteers took 4 capsules per day, 2 in the morning and 2 in the evening, on an empty stomach. Omega-3 index tests were again taken after 1 month. Table 4 shows the effect of xenon on the change of omega-3 index after three months of product intake in control and experimental groups.

**Table 4**

| | Omega-3 index | gas |
|---|---|---|
| control | 5,1 | - |
| control | 5,3 | - |
| experience 1 | 6,7 | xenon 5 vol.% |
| experience 1 | 6,9 | xenon 5 vol.% |
| experience 2 | 8,2 | xenon 20 vol.% |
| experience 2 | 8,4 | xenon 20 vol.% |
| experience 3 | 8,9 | xenon 250 vol.% |
| experience 3 | 8,8 | xenon 250 vol.% |
| experience 4 | 5,9 | krypton 5 vol.% |
| experience 4 | 6,0 | krypton 5 vol.% |
| experience 5 | 8,4 | krypton 24 vol.% |
| experience 5 | 8,1 | krypton 24 vol.% |
| experience 6 | 8,8 | krypton 70 vol.% |
| experience 6 | 9,1 | krypton 70 vol.% |

The results show the effect of xenon and krypton on the dose-dependent increase in Omega-3 index.

## Claims

1. A method for improving the digestibility of products using an inert gas, said method including a mixing the inert gas with a functional nutrition product, **characterized in that** the functional nutrition products used are a fat emulsion, oils, water-soluble ingredients used in manufacturing of nutrition products, dietary supplements, beverages, and **in that** the active ingredient is at least one gas from the group including xenon, krypton, the gas content in the nutrition product being from 5 ml/l to 2,8 l/l, or a mixture thereof in various ratios.

2. The method according to claim 1, **characterized in that**, once a gas supply stopped, the nutrition product is cooled, packaged in a hermetically sealed packaging selected from the group of metal, plastic, glass, gelatin capsules.

3. The method according to claim 1, **characterized in that** an overpressure of up to 2 atm is used to saturate the functional product with the inert gas.

4. The method according to claim 1, **characterized in that** the functional nutrition product is made in a liquid, soft, semi-soft form.
